Europäisches Patentamt

European Patent Office

Office européen des brevets

(19)

(11) Publication number: **0 196 761 B1**

# EUROPEAN PATENT SPECIFICATION

(12)

(45) Date of publication of patent specification: **10.07.91**   (51) Int. Cl.⁵: **A61L 2/04**

(21) Application number: **86301196.1**

(22) Date of filing: **20.02.86**

The file contains technical information submitted after the application was filed and not included in this specification

(54) **Method of virus-inactivating heat treatment of gamma-globulin.**

(30) Priority: **21.02.85 JP 33335/85**

(43) Date of publication of application:
**08.10.86 Bulletin 86/41**

(45) Publication of the grant of the patent:
**10.07.91 Bulletin 91/28**

(84) Designated Contracting States:
**BE CH DE FR GB LI NL SE**

(56) References cited:
**EP-A- 0 035 204**
**EP-A- 0 037 078**
**EP-A- 0 077 870**
**EP-A- 0 124 506**
**EP-A- 0 131 740**

(73) Proprietor: **GREEN CROSS CORPORATION**
**15-1, Imabashi-1-chome Higashi-ku Osaka-shi**
**Osaka 541(JP)**

(72) Inventor: **Hirao, Yutaka**
**14-1-606, Terauchi-2-chome**
**Toyonaka-shi(JP)**
Inventor: **Uriyu, Katuhiro**
**601-32, Nakatsumichi-3-chome**
**Daifuku Sakurai-shi(JP)**
Inventor: **Uemura, Yahiro**
**Mezon Hirakata 215 5-18, Mitsuyacho**
**Hirakata-shi(JP)**

(74) Representative: **Harrison, David Christopher et al**
**MEWBURN ELLIS & CO 2/3 Cursitor Street**
**London EC4A 1BQ(GB)**

## Description

This invention relates to a method of heat treatment of an aqeous solution containing γ-globulin. More particularly, it relates to a method of stabilizing the γ-globulin during heat treatment wherein a selected stabilizer is added to the aqueous solution of γ-globulin and no increase of the dimer or polymer content of γ-globulin (a measure of denaturation) nor increase in anticomplement activity is observed after low temperature pasteurization, eg. a treatment conducted at 60° C for 10 hours.

Preparations containing among the immunoglobulins of a plasma protein component, the particular protein gamma-globulin (IgG) as the principal ingredient have been widely used in the prevention and the treatment of various infectious diseases. However, in the past difficulties have been encountered when subjecting such preparations to heat-sterilization because they have a poor heat stability and they contain a wide variety of antibodies to various viruses and bacteria, the activity of which is liable to be lost.

However, when γ-globulin is prepared from a fraction of plasma protein, the possibility of contamination with viruses such as hepatitis viruses cannot be ignored completely. Accordingly, it is important that γ-globulin preparations are subjected beforehand to a heat treatment at 60° C for 10 hours, which treatment has been widely recognized in blood component preparation technology as a method for inactivating contaminating viruses.

However, when the treatment is conducted in a conventional aqueous solution such as physiological saline solution, the solution becomes turbid in a short time, most of the activity being lost and the protein molecules being denatured.

EP-A-0124506 describes a process for inactivating viruses present in solutions containing a wide variety of pathogenic proteins by heat treatment in the presence of ammonium sulphate as stabilizer. Glycine may be additionally present as an auxiliary protein stabilzer.

EP-A-0131740 describes a virus inactivation process in which a protein-containing solution is treated with a di- or trialkyl phosphate. The process is said to be combinable with a conventional heat treatment process carried out in the presence of a stabilizer for the protein.

EP-A-0035204 contains a general disclosure of the inactivation of a wide range of viruses present in a wide range of protein-containing media by heating in the presence of a wide range of polyols. An advantage is said to be that the inactivation process may be applied to blood plasma prior to fractionation. The specific disclosure refers only to the use of sucrose as stabilizer in the heat treatment of many protein-containing media, for example, immune serum globulin (Example 2).

EP-A-0077870 describes the inactivation by heat treatment of any virus present in a solution of factor VIII using as principal stabilizer a neutral amino acid, a mono- or oligosaccharide or a sugar alcohol and as auxiliary stabilizer a carboxylic or hydroxycarboxylic acid salt.

After extensive studies, the present inventors have found that the thermal stability of γ-globulin against heat treatment is markedly improved when at least one member (hereinafter referred to generically as a principal stabilizer) selected from glucose, mannitol and sorbitol is added to an aqueous solution containing γ-globulin prior to or at the time as the heat treatment of the solution for inactivating hepatitis viruses and that the thermal stability of γ-globulin is further enhanced when at least one member (hereinafter referred to generically as an auxiliary stabilizer) selected from a neutral inorganic acid salt, a surface active agent, and an organic carboxylic acid salt is added to the solution in addition to the principal stabilizer.

Thus, when the auxiliary stabilizer is present in a suitable amount, the anticomplementary activity of the γ-globulin is decreased as compared with that when the primary stabilizer alone is present.

Further, the heat treatment according to the method of this invention makes it possible to dissociate the dimer or polymer of γ-globulin contained in the aqueous γ-globulin solution into its monomer.

The aqueous solution containing γ-globulin to be heat-treated according to this invention may be an aqueous γ-globulin solution at any stage of purification ranging from an unpurified aqueous solution containing γ-globulin to a purified aqueous solution. However, an aqueous solution at a partially purified or purified stage is advantageously subjected to the heat treatment. The aqueous solution contains 0.1 to 30% (w/v) of protein (γ-globulin). The pH of the aqueous solution is generally 4.5 to 10, and is preferably adjusted to pH 6 to 8 with a suitable buffer solution.

The principal stabilizers added to the aqueous solution are glucose, mannitol and sorbitol. The amount of the principal stabilizer to be added is 10 to 100 g, preferably 40 to 100 g per 100 ml of aqueous γ-globulin solution.

Among the auxiliary stabilizers used in this invention, the neutral inorganic acid salts include, for example, the halides of alkali metals or alkaline earth metals such as sodium chloride, potassium chloride, and magnesium chloride. Their amount to be added is preferably 0.1 to 10 g per 100 ml of aqueous γ-globulin solution.

2

The organic carboxylic acid referred to in this invention is a compound comprising a hydrocarbon residue and a carboxyl substitute attached thereto. The hydrocarbon residue may be either saturated or unsaturated, and either chain-like (straight chain or branched chain) or cyclic. Examples of the hydrocarbon residue include an alkyl group and an aryl group (such as a phenyl group). The number of carboxyl groups in the organic carboxylic acid may be plural, but is preferably one or two. Further, the organic carboxylic acid may have a hydroxyl group. The organic acid has preferably about 3 to about 15 carbon atoms.

The kind of organic carboxylic acid salt is not particularly restricted so long as it is physiologically acceptable. Preferred examples thereof include alkali metal salts such as sodium salts and potassium salts and alkaline earth metal salts such as calcium salts. Particularly preferable are sodium salts and potassium salts. Specific examples of the organic acid salts include particularly alkali metal salts (sodium or potassium salt) of propanoic, butanoic, pentanoic, caprylic, caproic, malonic, succinic, glutaric, adipic, citric and mandelic acid. The amount of the organic carboxylic acid salt to be added is 1 to 30 g per 100 ml of the aqueous $\gamma$-globulin solution.

Examples of the surface active agents usable in the method of this invention include nonionic surface active agents such as alkylphenyl-polyoxyethylene having a molecular weight of 500 to 1,000 [for example, Triton (a registered trade mark) and Nonidet (a registered trade mark)], anionic surface active agents such as bile acid salts, for example sodium taurocholate, cationic surface active agents such as benzalkonium chloride, and polyhydric alcohols having surface activity such as a high molecular weight copolymer of propylene oxide having a molecular weight of 2,000 to 12,000 [for example, Pluronic (a registered trade mark) F68]. The amount to be added is preferably about 0.002 to about 0.05 g per 100 ml of the aqueous $\gamma$-globulin solution.

The heat treatment should be conducted at a sufficient temperature and for a sufficient time for inactivating contaminating viruses only. For example, it is conducted at 50 to 70°C, preferably at about 60°C, for 5 to 20 hours, preferably for 10 hours.

In order to examine the effect of the heat treatment according to this invention, the effect of heating in the presence of a principal stabilizer and that in the absence of the principal stabilizer were tested in the following manner on the infectivity of various viruses whose possible presence in $\gamma$-globulin preparations is apprehended. Thus, smallpox viruses, parotitis viruses, measles viruses, vesicular viruses, chikungunya viruses, polioviruses, coxsackie viruses, or echoviruses were added to a $\gamma$-globulin solution specimen, the resulting mixture was heat-treated at 60°C for 10 hours, and the remaining infectivity of the viruses was determined with the lapse of time. The infectivity was found to have vanished completely after 10 hours irrespective of the presence or the absence of the stabilizer. The result suggests that other viruses than those used above will lose their infectivity when heat-treated according to this invention.

After the above-mentioned heat treatment in the presence of the principal stabilzer according to this invention, the resulting product was examined for its appearance and properties and further subjected to the quantitative determination of dimer or polymer of $\gamma$-globulin, the determination of anticomplement activity, the determination of measles antibody titer, and the acute toxicity test. The results obtained reveal, as disclosed in the Example below, a decrease in the amount of the dimer or polymer and in the anticomplement activity of $\gamma$-globulin, but the retention of the antibody titer, showing that it gives a $\gamma$-globulin preparation exhibiting an extremely high safety and a high effectiveness in medical treatment.

The product thus obtained is in a liquid state, and when a highly purified $\gamma$-globulin has been used as the starting material, is dispensed in that form, or, when it has been derived from a crude product, it is after treated according to a known method of purification followed, as required, by dialysis or sterile filtration, so as to provide a solution containing 50 to 10,000 mg of $\gamma$-globulin depending on the desired package unit dosage. The method of its storage is not particularly restricted so long as a high temperature is avoided. However, it is particularly preferably stored at a temperature not higher than 30°C or, as desired, may be made into a lyophilized preparation.

The $\gamma$-globulin thus treated is then administered as it is or after a preparation treatment known per se, for example, when for injection use, after being diluted by or dissolved in or dialyzed against distilled water. The usual unit dosage administered is 2,500 to 5,000 mg/kg body weight in terms of $\gamma$-globulin for adults and 100 to 150 mg/kg body weight in terms of $\gamma$-globulin for infants.

The present invention is further explained by the following Examples, but it is not limited thereto.

In the Examples, in order to demonstrate the improved clarity of compositions prepared by the method of the invention, this was assayed in terms of absorbance, O.D. 600 nm.

The quantity of the dimer or the polymer was determined by means of high performance liquid chromatography.

The anticomplement activity was determined according to the method of Kabatt and Meyer [Experimental Immunochemistry, 225 (1961)] and the method of Nishioka and Okada [Men'eki no Seikagaku (Biochemistry of Immunity) 103, (1971); published by Kyoritsu Shuppan Co.]. Namely, a specimen was added to 100 units of complement and the number of units remaining in the resulting mixture was determined. The anticomplement activity were expressed in terms of the decreased units.

The measles antibody titer was determined by a hemagglutination inhibition test and expressed in terms of international units (IU/150 mg).

Example 1

Experiments were made to confirm the stabilizing effect achieved by a method according to this invention. The experiments were conducted with samples prepared by adjusting a solution of a $\gamma$-globulin containing about 30% of polymer to a concentration of 5%. After the addition of various principal stabilizers (the amount added being indicated in the Table 1), the sample was heat-treated at 60°C for 10 hours and then examined for the turbidity (O.D. 600 nm) of the solution, the quantity of polymer and the anticomplement activity. The results obtained revealed that the stability of $\gamma$-globulin in heating was improved by the addition of stabilizer (Table 1).

Further, the decrease of amount of polymer, particularly dimer, was confirmed.

Table 1

| Stabilizer | Amount added *1 | O.D. 600 nm | Polymer (%) | | Anticomplement activity (unit) |
|---|---|---|---|---|---|
| | | | Dimer | Polymer | |
| Control (before heating) | – | 0.024 | 33 | 2 | 54 |
| None (for comparison) | – | Turbid | –*2 | – | – |
| Glucose | 50 | 0.010 | 15 | 2 | 38 |
| Mannitol | 20 | 0.017 | 17 | 2 | 42 |

Note: *1: Amount (g) per 100 ml of 5% (w/v) γ-globulin solution.

*2: So much as cannot be determined.

Example 2

Glucose was added in various concentrations to a γ-globulin solution containing about 20% (w/v) of polymer and the concentration of γ-globulin in the resulting mixture was adjusted to 5% (w/v). The solution thus obtained was heat-treated at 60°C, and the value of O.D. 600 nm, the quantity of polymer, the anticomplement activity, and the measles antibody titer were determined with the lapse of time.

The system containing no glucose became turbid within one hour, showing the occurrence of denaturation. The systems containing added glucose showed increasing stability of γ-globulin with increasing amount of glucose added. The system to which 100 g of glucose had been added did not become turbid and showed no decrease in the measles antibody titer even after heated at 60°C for 10 hours. Further, the content of dimer decreased down to only 10% and the anticomplement activity also decreased down to 19 units (Table 2).

Table 2  Heat treatment at 60°C for 10 hours

| Amount of glucose added (g) | O.D. 600 nm | Polymer (%) | | Anticomplement activity (unit) | Measles antibody titer (IU) |
|---|---|---|---|---|---|
| | | Dimer | Polymer | | |
| Control (before heating) | 0.024 | 22 | 3 | 54 | 42 |
| None | Turbid | - *1 | - | - | - |
| 5 | Turbid | - | - | - | - |
| 25 | 0.040 | 15 | 30 | >50 | <10.5 |
| 50 | 0.010 | 13 | 3 | 36 | 21 |
| 75 | 0.004 | 12 | 2 | 28 | 40 |
| 100 | 0.004 | 10 | 2 | 19 | 40 |

Note: *1: So much as cannot be determined.

Example 3

In addition to the principal stabilizer, glucose, one or two auxiliary stabilizers selected from a neutral amino acid (glycine), a neutral salt (sodium chloride), an organic carboxylic acid salt (sodium citrate), and a surface active agent (Pluronic® F68), were added to a γ-globulin solution and the stability of the γ-globulin in the resulting solution after heat treatment at 60°C for 10 hrs was examined. The test was conducted with the same γ-globulin solution as in Example 1 but containing about 15% (w/v) of polymer.

Heat treatment at 60°C for 10 hours was conducted for each of the systems to which 75 g of glucose was added in common per 100 ml of γ-globulin solution and 5.8% (w/v) of sodium chloride, 5% (w/v) of glycine, 10% (w/v) of sodium citrate, 0.01% (w/v) of Pluronic® F68, or a combination of two auxiliary stabilizers, 5.8% (w/v) of sodium chloride and 0.01% (w/v) of Pluronic® F68, was added. The results obtained are shown in Table 3. The results reveal that the content of polymer and the anticomplement activity can be further decreased by the addition of the auxiliary stabilizers.

Table 3

| Auxiliary stabiliser (Referred to in Example 4) | Amount added (g) | O.D. 600 nm | Polymer (%) | | Anti-complement activity (unit) | Measles antibody titer (IU) |
|---|---|---|---|---|---|---|
| | | | Dimer | Polymer | | |
| Control (before heating) | – | 0.004 | 15 | 2 | 44 | 42 |
| None (A) | – | 0.004 | 8 | 1 | 28 | 40 |
| Sodium chloride (B) | 5.8 | 0.004 | 5 | 1 | 18 | 42 |
| Sodium citrate (C) | 10 | 0.004 | 8 | | 24 | 38 |
| Pluronic® F68 (D) | 0.01 | 0.004 | 6 | 1 | 13 | 40 |
| Sodium chloride / Pluronic® F68 (E) | 5.8 / 0.01 | 0.004 | 6 | 1 | 12 | 41 |

Example 4

Acute toxicity tests were conducted by way of a safety test.
Samples A, B, C, D and E which had been heat-treated at 60°C for 10 hours in Example 3 were

8

dialyzed thoroughly against sterile physiological saline, and then administered respectively to mice in groups of five through the tail vein in a total amount of 0.5 ml and 1.0 ml per one animal. No abnormality was found after 7 days of observation.

## Claims

1. A method of virus-inactivating heat treatment of an aqueous γ-globulin solution containing 0.1 to 30% (w/v) inclusive of γ-globulin in terms of protein and having a pH of from 4.5 to 10 inclusive, which method comprises adding to the aqueous solution at least one stabilizer in an amount of from 10 to 100g, inclusive per 100 ml of the solution, which stabilizer is selected from glucose, mannitol and sorbitol, and heating the aqueous solution at a temperature of from 50° to 70°C inclusive for a period sufficient substantially to free the aqueous γ-globulin solution from infectivity of a virus.

2. A method according to claim 1, wherein the amount of the stabilizer is from 40 to 100g inclusive per 100 ml of the solution.

3. A method of virus-inactivating heat treatment of an aqueous γ-globulin solution containing 0.1 to 30% (w/v) inclusive of γ-globulin in terms of protein and having a pH of from 4.5 to 10 inclusive, which method comprises adding to the aqueous solution (1), in an amount of from 10 to 100g inclusive per 100 ml of the solution, a primary stabilizer selected from glucose, mannitol and sorbitol, and (2) an auxiliary stabilizer selected from a neutral inorganic salt, an organic carboxylic acid having from 3 to 10 carbon atoms inclusive and a surface active agent, and heating the aqueous solution at a temperature of from 50°C to 70°C inclusive for a period sufficient to free the aqueous γ-globulin solution from infectivity of a virus.

4. A method according to claim 3, wherein the auxiliary stabilizer is an organic carboxylic acid and is present in an amount of from 1 to 30 g inclusive per 100 ml of the solution.

5. A method according to claim 3, wherein the auxiliary stabilizer is an inorganic acid salt and is present in an amount of from 0.1 to 10 g inclusive per 100 ml of the solution.

6. A method according to claim 3, wherein the auxiliary stabilizer is a surface active agent and is present in an amount of from 0.002 to 0.05 g inclusive per 100 ml of the solution.

## Revendications

1. Procédé d'inactivation de virus par traitement thermique d'une solution aqueuse de γ-globuline contenant de 0,1 à 30 % (p/v) inclus de protéine de γ-globuline, et ayant un pH de 4,5 à 10 inclus, dans lequel on ajoute dans la solution aqueuse, au moins un stabilisant selon une quantité de 10 à 100 g inclus pour 100 ml de la solution, ce stabilisant étant choisi parmi le glucose, le mannitol et le sorbitol, et on chauffe la solution aqueuse à une température de 50 à 70 °C inclus pendant un temps suffisant pour pratiquement supprimer l'infectivité d'un virus dans la solution aqueuse de γ-globuline.

2. Procédé selon la revendication 1, dans lequel la quantité du stabilisant est de 40 à 100 g inclus pour 100 ml de la solution.

3. Procédé d'inactivation de virus par traitement thermique d'une solution aqueuse de γ-globuline contenant de 0,1 à 30 % (p/v) inclus de protéine de γ-globuline, et ayant un pH de 4,5 à 10 inclus, selon lequel on ajoute dans la solution aqueuse, (1) un stabilisant primaire choisi parmi le glucose, le mannitol et le sorbitol, selon une quantité de 10 à 100 g inclus pour 100 ml de la solution, et (2) un stabilisant auxiliaire choisi parmi un sel inorganique neutre, un acide carboxylique organique comportant de 3 à 10 atomes de carbone inclus et un agent tensio-actif, et on chauffe la solution aqueuse à une température de 50 à 70°C inclus pendant un temps suffisant pour pratiquement supprimer l'infectivité d'un virus dans la solution aqueuse de γ-globuline.

4. Procédé selon la revendication 3, dans lequel le stabilisant auxiliaire est un acide carboxylique organique incorporé selon une quantité de 1 à 30 g inclus pour 100ml de la solution.

5. Procédé selon la revendication 3, dans lequel le stabilisant auxiliaire est un sel d'acide inorganique incorporé selon une quantité de 0,1 à 10 g inclus pour 100 ml de la solution.

6. Procédé selon la revendication 3, dans lequel le stabilisant auxiliaire est un agent tensio-actif incorporé selon une quantité de 0,002 à 0,05 g inclus pour 100 ml de la solution.

**Ansprüche**

1. Verfahren zur Virus-inaktivierenden Hitzebehandlung einer wäßrigen $\gamma$-Globulinlösung, die 0,1 bis 30 % (Gew./V) $\gamma$-Globulin, ausgedrückt als Protein, enthält und die einen pH-Wert von 4,5 bis 10 aufweist, wobei das Verfahren die Zugabe von mindestens einem Stabilisator in einer Menge von 10 bis 100g/100 ml der Lösung zu der wäßrigen Lösung, welcher ausgewählt ist aus Glucose, Mannit und Sorbit, und Erhitzen der wäßrigen Lösung auf eine Temperatur von 50°C bis 70°C während einer Zeitspanne, die ausreicht, um die wäßrige $\gamma$-Globulinlösung im wesentlichen von der Infektiosität eines Virus zu befreien, umfaßt.

2. Verfahren nach Anspruch 1, wobei die Menge des Stabilisators von 40 bis 100 g/100 ml der Lösung beträgt.

3. Verfahren zur Virus-inaktivierenden Hitzebehandlung einer wäßrigen $\gamma$-Globulinlösung, die 0,1 bis 30 % (Gew./V.) $\gamma$-Globulin, ausgedrückt als Protein, enthält und die einen pH-Wert von 4,5 bis 10 aufweist, wobei das Verfahren die Zugabe von (1), einem primären Stabilisator, ausgewählt aus Glucose, Mannit und Sorbit, in einer Menge von 10 bis 100 g/100 ml der Lösung, und (2) einem Hilfsstabilisator, ausgewählt aus einem neutralen anorganischen Salz, einer organischen Carbonsäure mit 3 bis 10 Kohlenstoffatomen und einem grenzflächenaktiven Mittel, zu der wäßrigen Lösung und Erhitzen der wäßrigen Lösung auf eine Temperatur von 50°C bis 70°C während einer Zeitspanne, die ausreicht, um die wäßrige $\gamma$-Globulinlösung im wesentlichen von der Infektiosität eines Virus zu befreien, umfaßt.

4. Verfahren nach Anspruch 3, wobei der Hilfsstabilisator eine organische Carbonsäure ist und in einer Menge von 1 bis 30 g/100 ml der Lösung vorliegt.

5. Verfahren nach Anspruch 3, wobei der Hilfsstabilisator ein anorganisches Salz ist und in einer Menge von 0,1 bis 10 g/100 ml der Lösung vorliegt.

6. Verfahren nach Anspruch 3, wobei der Hilfsstabilisator ein grenzflächenaktives Mittel ist und in einer Menge von 0,002 bis 0,05 g/100 ml der Lösung vorliegt.